Europäisches Patentamt

European Patent Office

Office européen des brevets

(19)

(11) Numéro de publication: **0 093 032**
**A1**

(12) **DEMANDE DE BREVET EUROPEEN**

(21) Numéro de dépôt: **83400719.7**

(51) Int. Cl.³: **G 06 F 15/20**

(22) Date de dépôt: **12.04.83**

(30) Priorité: **23.04.82 FR 8207005**

(43) Date de publication de la demande:
**02.11.83 Bulletin 83/44**

(84) Etats contractants désignés:
**AT BE CH DE GB IT LI LU NL SE**

(71) Demandeur: **S.E.R.E.M.**
**Zone d'Activités des Godets 37, rue des Petits Ruisseaux**
**F-91370 Verrieres le Buisson(FR)**

(72) Inventeur: **L'Henoret, Louis**
**115, Avenue François Molé**
**F-92160 Antony(FR)**

(74) Mandataire: **Marquer, Francis**
**35, Avenue Victor Hugo Résidence Chamfleury**
**F-78180 Voisins-le-Bretonneux(FR)**

(54) Dispositif de mise en mémoire numérique et de visualisation, d'un électrocardiogramme ou autre signal d'origine physiologique ou physique, en vue de son contrôle, de son traitement et de son stockage.

(57) Dispositif de mise en mémoire numérique et de visualisation d'un électrocardiogramme ou autre signal d'origine physiologique ou physique, en vue de son contrôle, de son traitement et de son stockage, caractérisé en ce qu'il comporte deux mémoires numériques (1, 2), des moyens (3 à 12) d'introduire et d'enregistrer l'information contenue dans un support d'enregistrement ou fournie en temps réel par un capteur quelconque et correspondant à une durée prédéterminée d'enregistrement (par exemple vingt-quatre heures) successivement dans la première (1) et la seconde (2) mémoires; et des moyens (13-16; 20-21; 23-27) de visualiser sur un écran principal l'information contenue dans chacune des mémoires (1-2), pendant le temps d'acquisition de l'information par l'autre mémoire, ce processus d'acquisition et de lecture simultanée se déroulant un nombre de fois suffisant pour que le contenu total de la bande magnétique ait été visualisé sur l'écran principal.

./...

FIG 1

- 1 -

## Dispositif de mise en mémoire numérique et de visualisation d'un électrocardiogramme ou autre signal d'origine physiologique ou physique, en vue de son contrôle, de son traitement et de son stockage.

On connaît des appareils effectuant l'enregistrement de deux ou plusieurs voies ECG, à la fois sur une bande de papier en défilement permanent et sur une cassette qui contient par exemple vingt quatre heures d'enregistrement.

Un appareil connu comprend des moyens de lecture, soit en temps réel, soit rapide (en douze minutes par exemple) de la totalité de la bande magnétique correspondant à vingt-quatre heures d'enregistrement et des moyens d'analyse automatique du signal ECG, en vue de reconnaître et de compter différents accidents qui se produisent de façon périodique selon le rythme propre du cœur étudié. Lesdits moyens de lecture sont reliés à un dispositif de visualisation du tracé sur un écran, soit en régime déclenché, c'est-à-dire avec visualisation d'un ou plusieurs complexes QRS dont on pourra ainsi observer l'évolution dans le temps, soit en régime de mémorisation figée, dans lequel une portion du signal est figée sur l'écran pendant un certain temps.

- 2 -                    0093032

Les moyens d'analyse automatique peuvent comporter des moyens d'établir des courbes représentatives de tendances propres au tracé considéré telles que, par exemple, l'évolution de la fréquence cardiaque, de la densité des extra-systoles, et autres. Comme le médecin ne fait pas entièrement confiance aux résultats de l'analyse automatique, l'appareil comprend en outre des moyens d'établir le tracé continu du signal ECG pendant toute la durée de l'enregistrement. Ce tracé permet le contrôle visuel permanent des informations fournies par l'analyse. Dans l'appareil connu mentionné ci-dessus, cette sortie analogique s'effectue sur papier photographique, à la vitesse de lecture accélérée, c'est-à-dire qu'une heure d'enregistrement est inscrite en 30 secondes et forme 60 lignes sur le papier. A cet effet, on utilise un enregistrement à fibres optiques et l'opérateur, peut s'il le désire, inscrire en surbrillance les anomalies détectées par les moyens de lecture.

Un inconvénient de ce dispositif connu de contrôle de l'analyse est son coût de fabrication et surtout de mise en œuvre relativement élevé.

La présente invention se propose de réaliser le contrôle visuel de l'analyse et, d'une façon plus générale, la mise en mémoire de l'information et sa visualisation à l'aide de moyens peu onéreux, faciles à mettre en œuvre en un temps relativement bref, et offrant des possibilités multiples de contrôle et de stockage de l'information.

Suivant l'invention, ces résultats sont obtenus au moyen d'un dispositif comportant deux mémoires numériques, des moyens d'introduire et d'enregistrer l'information contenue dans un support d'enregistrement ou fournie en temps réel par un capteur quelconque et correspondant à une durée prédéterminée d'enregistrement (par exemple vingt-quatre heures) successivement dans la première et la seconde mémoires ; et des moyens de visualiser sur un écran principal l'information contenue dans chacune des mémoires, pendant le temps d'acquisition de l'information par l'autre mémoire, ce processus d'acquisition et de lecture simultanée se déroulant un nombre de fois suffisant pour que le contenu total de la bande magnétique ait été visualisé sur l'écran principal.

Suivant une particularité de l'invention, le dispositif de contrôle comporte en outre une mémoire auxiliaire dans laquelle les accidents de l'électrocardiogramme relevés par le praticien, au cours de la visualisation sur l'écran principal, sont mémorisés après sélection à l'œil et des moyens de visualiser les accidents ainsi sélectionnés et mémorisés, sur un écran auxiliaire, lesdits moyens de visalisation auxiliaire étant agencés pour obtenir un effet de loupe.

Dans un mode d'exécution préféré, lesdits moyens d'enregistrement, dans les deux mémoires numériques, sont utilisés pour alimenter en information les moyens d'analyse automatique du signal ECG, des moyens étant prévus pour lire vers les moyens d'analyse le contenu de chaque adresse de chacune des deux mémoires, juste avant son effacement en vue de l'acquisition qui suit.

Dans une forme d'exécution plus particulière, l'appareil étant du type connu dans lequel on effectue l'enregistrement magnétique d'une porteuse modulée en fréquence par le signal ECG, la lecture dudit enregistrement magnétique comportant une démodulation synchrone au moyen d'un convertisseur fréquence/tension, et la fréquence de ladite porteuse étant définie par une horloge à quartz, l'invention prévoit d'utiliser ladite fréquence d'horloge pour effectuer l'échantillonnage des signaux nécessaires à leur mise en mémoire numérique et pour fournir les signaux de synchronisation nécessaires à l'enregistrement dans lesdits mémoires.

Il en résulte que le signal mis en mémoire et visualisé sur les écrans peut être en temps réel, même s'il se produit des dérives de la vitesse d'enregistrement ou de lecture magnétiques.

D'autres particularités, ainsi que les avantages de l'invention, apparaîtront clairement à la lumière de la description ci-après.

Au dessin annexé :

La figure 1 est un schéma de principe de la partie "mémoires et enregistrement" d'un dispositif de contrôle conforme à un mode d'exécution préféré de l'invention ; et

La figure 2 représente schématiquement la partie "lecture" d'un tel dispositif.

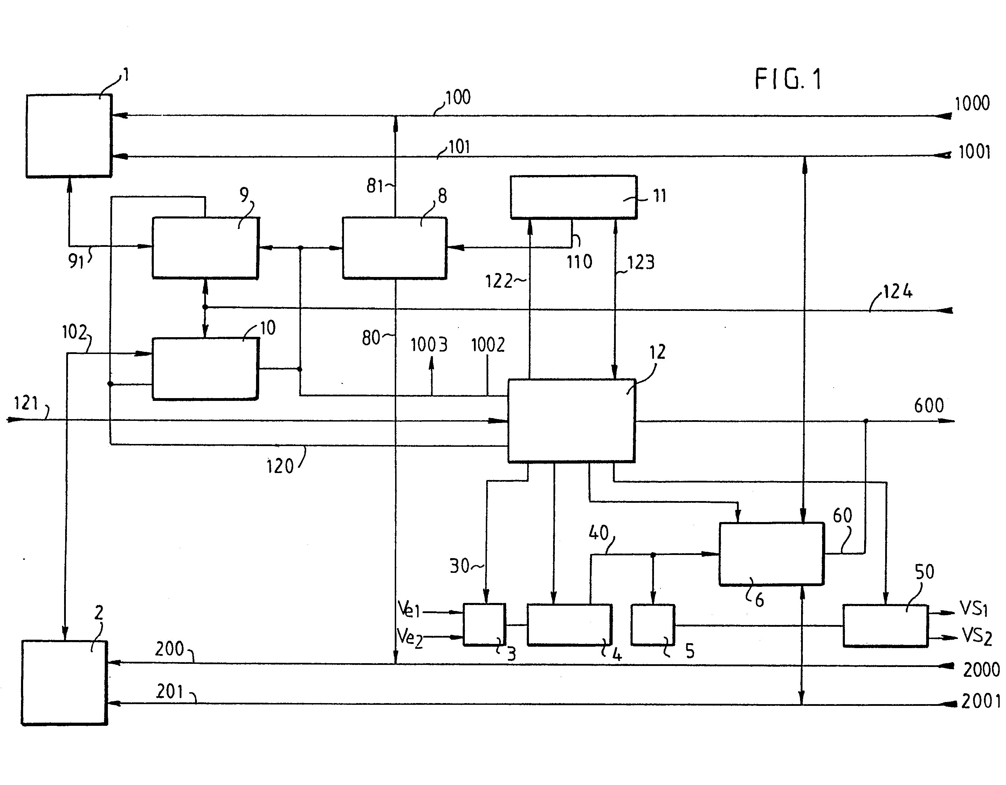

A la figure 1, on a représenté deux mémoires numériques 1 et 2 ayant chacune un bus d'adresses 100, respectivement 200 et un bus de données 101, respectivement 201. Chaque mémoire a par exemple une capacité de 128 kilooctets, dont 2048 x 60 adresses seront utilisées pour contenir un nombre correspondant de points échantillonnés du signal ECG et les 4 x 2048 autres seront utilisés comme réserve, à des fins qui seront expliquées plus loin.

On supposera, dans ce qui suit, que le signal fourni par les moyens de lecture, non figurés, que comporte l'appareil, comprend deux voies ECG $Ve_1$ et $Ve_2$. Celles-ci sont appliquées à un multiplexeur d'entrées 3 qui les transmet à un convertisseur analogique-numérique 4. La sortie de celui-ci est reliée, d'une part, à un convertisseur numérique-analogique 5, lui-même suivi d'un démultiplexeur 50, d'autre part, à un multiplexeur de données 6 qui aiguille, dans les deux sens, les données vers le bus 101 ou le bus 201, comme on l'expliquera dans la suite.

De même, un multiplexeur d'adresses 8 est relié aux bus d'adresses respectifs 100 et 200.

Les contrôles d'entrées et de sorties dans les mémoires s'effectuent respectivement au moyen d'un multiplexeur 9 pour la mémoire 1 et d'un multiplexeur 10 pour la mémoire 2, tandis que le comptage des adresses à l'enregistrement s'effectue au moyen d'un compteur 11. Une base de temps 12 envoie aux différents organes les signaux de synchronisation nécessaires à leur fonctionnement, et reçoit elle-même une fréquence de référence de vitesse ou de temps sur son entrée 121. Cette fréquence est avantageusement dérivée de celle de l'horloge du dispositif d'enregistrement magnétique que comporte l'appareil, comme il a été dit plus haut.

Pour expliquer le fonctionnement du dispositif, on partira arbitrairement de l'instant où va s'effectuer une inscription à une adresse paire $x_{2i}$ de la mémoire 2, en supposant par exemple que les adresses paires des deux mémoires soient affectées à la 1ère voie ECG, les adresses impaires étant affectées à la 2ème voie.

Un échantillon du signal ECG de la 1ère voie est fourni par le multiplexeur 3, lui-même synchronisé par un signal fourni à son entrée 30 par la base de temps 12. Cet échantillon est transformé en valeur numérique par le convertisseur 4. Le multiplexeur 6 reçoit, à cet instant, sur son entrée 60, un signal de la base de temps qui a pour effet d'aiguiller ladite valeur numérique vers le bus 201. Par ailleurs, le compteur d'adresses 11 envoie sur sa sortie 110 un signal au multiplexeur d'adresses 8 et ce dernier, par sa sortie 80, envoie sur le bus 200 l'information d'adresses $x_{2i}$.

Le multiplexeur 8 commande l'envoi d'une information d'adresses sur sa sortie 80 et non sur sa sortie 81, car il a reçu un ordre approprié à cet effet de la base de temps 12 par le fil 1002 de commande de direction d'adresses.

Les multiplexeurs 9 et 10, d'une part, reçoivent par le fil 120 une information de la base de temps qui indique que les mémoires doivent travailler en enregistrement, d'autre part, sont reliés aux mémoires respectives par des fils 91, 102, de façon à indiquer les instants où une opération d'enregistrement ou de lecture peut être prise en compte : il faut, en particulier, pour effectuer un enregistrement, que le convertisseur 4 ait achevé sa conversion.

Lorsque l'inscription à l'adresse $x_{2i}$ est terminée, la base de temps fait progresser le compteur 11 à l'adresse $x_{2i+1}$ (par le fil 122) et l'inscription de l'échantillon suivant, qui provient du signal ECG de la seconde voie, s'effectue suivant le processus qui vient d'être décrit.

Lorsque 60 x 2048 points ont été enregistrés dans la mémoire 2, le compteur 11 en informe la base de temps par le fil 23 et celle-ci, commute le multiplexeur 6 sur le bus 101 et le multiplexeur 8 sur le bus 100, de façon que les échantillons suivants du signal soient maintenant enregistrés dans la mémoire 1.

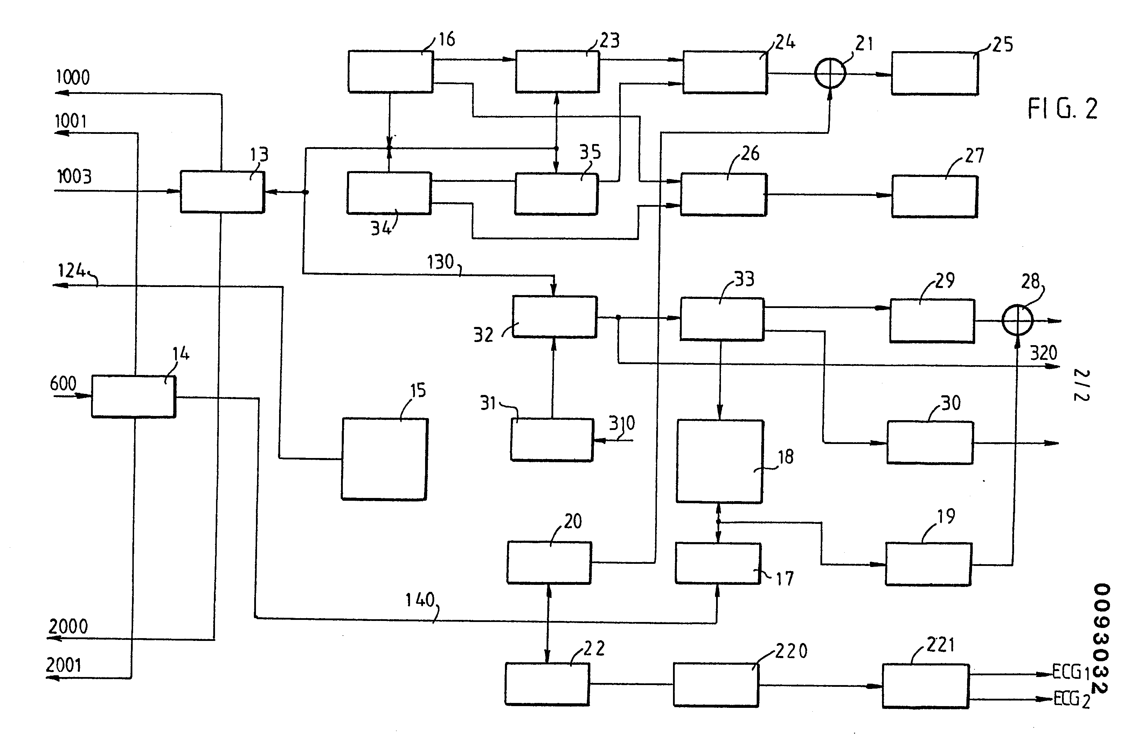

En outre, la base de temps commande, par l'intermédiaire des multiplexeurs 9 et 10, le passage de la mémoire 2 en régime de lecture principale et l'information qui vient d'y être enregistrée est alors visualisée sur l'écran principal, de la manière qui sera expliquée en se référant à la figure 2. Cette lecture dure le même temps que le remplissage de la mémoire 1 par les 60 x 2048 points suivants.

Ensuite, la mémoire 1 passe de la même manière en régime de lecture et visualisation, tandis que la mémoire 2 repasse en régime d'acquisition et d'enregistrement.

Dans l'exemple considéré, l'information enregistrée dans l'ensemble des deux mémoires correspond à 2 x 60 lignes de 2048 points, c'est-à-dire, à un quart d'heure d'enregistrement des deux voies.

Pour 24 heures, il faudra donc recommencer le processus d'enregistrement de chaque mémoire 96 fois. Si ce processus dure 7,5 sec., la durée totale de visualisation sera de 12 mn.

Lorsque l'une des mémoires, 2 par exemple, est en régime d'inscription, il est néanmoins systématiquement communiqué au multiplexeur 10, par le fil 100, un ordre de lecture de la donnée qui se trouve dans l'adresse où l'on s'apprête à en enregistrer une nouvelle. Cette donnée lue sur le bus 201 avant l'inscription de la nouvelle donnée est alors transmise, par le multiplexeur 6, vers le convertisseur 5 et démultiplexée par 50 pour sortir en $VS_1$ ou $VS_2$ (suivant qu'il

s'agit d'un signal ECG de la lère ou de la seconde voie). Autrement dit, on dispose, sur ces sorties, du signal ECG retardé du temps nécessaire à l'enregistrement complet des deux mémoires, c'est-à-dire d'une demi-heure dans l'exemple considéré, lorsque l'on travaille sur les deux voies.

L'ensemble des deux mémoires joue ainsi le rôle d'une ligne à retard, dont la sortie $VS_1$ ou $VS_2$ est alors reliée au dispositif d'analyse. La visualisation du contenu de chaque mémoire s'effectuant finalement avant la sortie des signaux $VS_1$ et $VS_2$, il est possible de supprimer les parasites qui peuvent affecter lesdits signaux, et dont la visualisation a montré l'existence. Il suffit, pour cela, d'appliquer le crayon optique sur les portions de lignes de l'écran principal (figure 2) qui comportent les parasites. Ces portions seront ainsi effacées d'une manière que l'on expliquera dans la suite en se référant à la figure 2 et ne seront pas prises en compte dans les résultats de l'analyse.

On va maintenant décrire, en se référant à la figure 2, le dispositif de lecture des mémoires 1 et 2.

Les bornes 1000 et 2000 de la figure 1 sont reliées à un multiplexeur d'adresses 13, tandis que les bornes 1001 et 2001 sont reliées à un multiplexeur de données 14. Une base de temps de lecture 15 est reliée au fil 124 de contrôle des mémoires en lecture de la figure 1. La borne 600 du fil de commande de direction des données de la figure 1 est reliée au multiplexeur 14 et la borne 1003 du fil de commande de direction des adresses de la figure 1 est reliée au multiplexeur 13.

Un compteur principal 16 a pour fonction de provoquer la lecture successive des données contenues dans les différentes adresses de la mémoire en cours de lecture et leur recirculation permanente en vue de leur visualisation sur l'écran principal.

Le compteur principal fait progresser l'adresse de lecture, par exemple dans la mémoire 1, au moyen du multiplexeur d'adresses 13. Les données lues dans la mémoire 1 sont dirigées par le multiplexeur 14, et un bus de données en lecture 140, vers un multiplexeur 17 qui peut les transmettre, d'une part, à une mémoire auxiliaire 18, d'autre part, à un convertisseur numérique analogique auxiliaire 19. Ces données sont par ailleurs transmises à un convertisseur numérique analogique principal 20, qui les transmet à un circuit de sommation 21, et elles peuvent encore être transmises à un deuxième convertisseur numérique analogique auxiliaire 22, suivi d'un démultiplexeur 220 et d'un ensemble de filtrage 221.

Les bits de poids forts appropriés du compte du compteur 16 passent dans un convertisseur numérique analogique 23 agencé pour fournir un signal de balayage vertical de trame, correspondant à 60 lignes et transmis par un multiplexeur 24, à l'amplificateur de déflexion verticale 25 du tube cathodique principal que comporte le dispositif. Ainsi, le signal appliqué à l'amplificateur 25 est la somme du signal analogique ECG issu du convertisseur principal 20 et du signal de trame.

Par ailleurs, un générateur de rampe 26 fournit à l'amplificateur de déflexion horizontale 27 du tube cathodique, un signal de balayage de ligne, produit à partir de l'information appropriée fournie par le compteur 16. On obtient ainsi la visualisation des 60 lignes de 2048 points que chaque mémoire contient pour chaque voie.

Le dispositif de lecture de la figure 2 comprend en outre des moyens de transférer, sur un tube cathodique auxiliaire, dont le balayage vertical est commandé par circuit sommateur 28 qui reçoit la sortie d'un convertisseur numérique analogique 29 agencé pour fournir un signal de trame à 8 lignes et la sortie du convertisseur 19 déjà mentionné, et dont le balayage horizontal est commandé par un générateur de rampe 30, d'une ligne sélectionnée sur l'écran principal.

L'opérateur effectue cette sélection au moyen d'un crayon optique qui détecte le passage du spot du tube cathodique à l'endroit pointé sur l'écran principal et est relié à un compteur 31, lequel mémorise l'adresse correspondant audit spot. Ce compteur 31 est relié à un comparateur 32 qui fournit un signal de synchronisation, d'une part, à l'entrée de surbrillance du tube cathodique principal, d'autre part, à un compteur 33 d'adressage de la mémoire auxiliaire, lorsqu'il y a coïncidence entre l'adresse pointée et l'adresse de lecture dans la mémoire de masse, fournie par le bus général d'adresses de lecture 130 relié à la sortie du multiplexeur 13.

Le compteur 33, qui reçoit la même fréquence d'horloge que le compteur principal 16, compte 2048 points à partir de l'adresse pointée, et adresse ainsi la mémoire auxiliaire à 2048 adresses 18. Les données correspondantes arrivant sur le bus de lecture 140 sont ainsi enregistrées dans la mémoire auxiliaire, qui contiendra une ligne de l'écran principal, positionnée à partir de l'endroit pointé, lequel sera matérialisé par une surbrillance sur l'écran principal.

Lorsque la mémoire 18 est enregistrée, la base de temps 15 commande son passage en régime de lecture, et les données, converties par 19, sont appliquées sur la sortie de balayage vertical du tube cathodique auxiliaire, en même temps qu'un signal de trame fourni par le convertisseur 29. Le générateur de rampe 30 est synchronisé par le compteur 33. En définitive, une ligne de l'écran principal est ainsi transférée sur huit lignes de l'écran auxiliaire, ce qui procure un effet de loupe pour l'examen des accidents sélectionnés.

Il est possible de transférer l'information ainsi visualisée sur l'écran auxiliaire dans la zone de 8 Kilooctets réservée dans celle des mémoires 1 ou 2 en cours de lecture. A cet effet, par exemple, la mémoire 1 est mise temporairement en régime d'écriture et on utilise un compteur d'adresses supplémentaire 34 pour effectuer l'écriture. Le compteur 16 étant alors déconnecté tout en continuant à compter, le compteur 34 opère le transfert, dans la zone réservée de la mémoire 1 de la totalité ou d'une fraction de la ligne sélectionnée. Le compteur 34, de capacité 8 kilooctets, est par ailleurs relié à un convertisseur numérique analogique supplémentaire 35 agencé pour engendrer un signal de balayage vertical correspondant à 16 lignes, lequel est transmis par le multiplexeur 24 à l'amplificateur de déflexion verticale 25. Le compteur de stockage 34, fournit en outre un signal de synchronisation sur 16 lignes, appliqué au générateur de rampe 26.

Lorsqu'aucune information n'a encore été enregistrée dans la zone réservée de la mémoire 1, le compteur 34 est au zéro. Il est en synchronisme exact avec le compteur 16 et va compter jusqu'à 2048 si l'on désire transférer une ligne complète de l'information sélectionnée par le crayon optique. Ce comptage commence au moment où la mémoire auxiliaire 18 est à l'adresse 0. Les multiplexeurs 17 et 14 transfèrent alors les données contenues dans la mémoire auxiliaire sur le bus 101, tandis que le compteur 34, par l'intermédiaire du multiplexeur 13, fournit sur le bus 100 les adresses de la zone réservée de la mémoire 1 où se fait l'inscription. Lorsque le transfert de la ligne ou de la portion de ligne sélectionnée est terminé, la mémoire 1 est remise en régime de lecture et c'est de nouveau le compteur 16 qui a continué à compter, qui reprend l'adressage, pour effectuer la visualisation sur l'écran principal. Le compteur 34 conserve son état jusqu'à ce que l'on désire effectuer un nouveau transfert dans la zone réservée de la mémoire de masse aux mémoires 1 et 2.

Il est possible de visualiser sur l'écran principal, avec effet de loupe, l'information inscrite en zone réservée de la mémoire de masse. Cette fonction est remplie par le compteur 34 et le convertisseur 35, en coopération avec les organes 24 à 27 et par 14 - 20 - 21. Lorsque le dispositif d'analyse est branché aux sorties $VS_1$, $VS_2$ de la figure 1, on a vu qu'il était possible d'effacer les portions de lignes de l'écran principal qui seraient affectées de parasites.

A cet effet, l'opérateur appose le crayon optique au début desdites portions ; la base de temps fait passer la mémoire de masse en écriture et envoie des zéros sur les groupes de 7 bits correspondant au signal utile dans les adresses correspondant à la partie à effacer et des uns sur les huitièmes bits correspondants, qui se trouvent toujours à zéro dans un signal ECG normal.

De cette façon, lors de l'analyse, le dispositif d'analyse, par la détection de bits positionnés à 1, recevra une information lui interdisant tout traitement.

Pendant la lecture normale des mémoires, ou encore pendant la lecture des accidents en zone réservée, les données sont accessoirement transmises par les circuits 22 -220 - 221, et deux signaux EG1 en temps réel apparaîssent sur les sorties ECG1, EGC2, figure 2, auxquelles peut être branché un électrocardiographe traditionnel, avec défilement du papier à la vitesse de 25 mm par sec., pour obtenir une représentation classique de l'ECG.

On notera par ailleurs que l'écran principal peut être photographié, page par page, au cours de la visualisation, au moyen d'un appareil photographique à développement instantané ou d'une caméra, en vue d'un stockage sur microfiches.

Il va de soi que diverses modifications pourront être apportées au dispositif décrit et représenté, sans s'écarter de l'esprit de l'invention.

En particulier, le signal d'entrée $Ve_1$ $Ve_2$, etc... pourrait provenir directement d'une acquisition en temps réel, une fréquence d'échantillonnage appropriée étant alors appliquée à la base de temps 12. En réanimation, il serait intéressant de visualiser ainsi, dans la salle de contrôle central, les électrocardiogramme ou autres signaux physiologiques relevés sur un ou plusieurs patients à surveiller. Ces électrocardiogrammes seront ainsi figés sur l'écran principal pendant toute la durée de l'acquisition du contenu d'une mémoire.

On notera que le dispositif de l'invention pourra être utilisé pour la visualisation et le traitement de signaux électriques d'origine quelconque.

Revendications de brevet

1. Dispositif de mise en mémoire numérique et de visualisation d'un électrocardiogramme ou autre signal d'origine physiologique ou physique, en vue de son contrôle, de son traitement et de son stockage, caractérisé en ce qu'il comporte deux mémoires numériques (1, 2), des moyens (3 à 12) d'introduire et d'enregistrer l'information contenue dans un support d'enregistrement ou fournie en temps réel par un capteur quelconque et correspondant à une durée prédéterminée d'enregistrement (par exemple vingt-quatre heures) successivement dans la première (1) et la seconde (2) mémoires ; et des moyens (13-16 ; 20-21 ; 23-27) de visualiser sur un écran principal l'information contenue dans chacune des mémoires (1-2), pendant le temps d'acquisition de l'information par l'autre mémoire, ce processus d'acquisition et de lecture simultanée se déroulant un nombre de fois suffisant pour que le contenu total de la bande magnétique ait été visualisé sur l'écran principal.

2. Dispositif selon la revendication 1, caractérisé en ce qu'il comporte en outre une mémoire auxiliaire (18) dans laquelle les accidents de l'électrocardiogramme relevés par le praticien au cours de la visualisation sur l'écran principal sont mémorisés, après sélection à l'œil et des moyens (19 et 28-33) de visualiser les accidents ainsi sélectionnés et mémorisés, sur un écran auxiliaire, lesdits moyens de visalisation auxiliaire étant agencés pour obtenir un effet de loupe.

3. Dispositif selon la revendication 1 ou 2, caractérisé en ce que lesdits moyens d'enregistrement (3-12), dans les deux mémoires numériques (1-2), sont utilisés pour alimenter en information les moyens d'analyse automatique du signal ECG, des moyens (5-50) étant prévus pour lire vers les moyens d'analyse ($VS_1$-$VS_2$) le contenu de chaque adresse de chacune des deux mémoires (1-2), juste avant son effacement en vue de l'acquisition qui suit.

4. Dispositif selon l'une des revendications 1 à 3 caractérisé en ce que, une fréquence d'horloge étant enregistrée sur la bande magnétique, lesdits moyens (3 à 12) d'introduire et d'enregistrer l'information, comprennent des moyens (30) d'échantillonner ladite information à ladite fréquence d'horloge et des moyens (12) de synchroniser les opérations d'enregistrement sur ladite fréquence.

5. Dispositif selon l'une des revendications 1 à 4, caractérisé en ce que lesdits moyens de visualisation comprennent un compteur principal d'adresses (16), un premier convertisseur numérique analogique principal (23) relié au compteur principal (16) pour fournir des signaux de balayage de trame de l'écran principal, un générateur de rampes relié au compteur principal (16) pour fournir des signaux de balayage de ligne de l'écran principal ; un second convertisseur numérique analogique principal (20) recevant les données lues dans l'une des deux mémoires numériques (1-2) et un circuit de sommation (21) des signaux de balayage de trame et des signaux de sortie du second convertisseur numérique analogique (20).

6. Dispositif selon la revendication 2, caractérisé en ce que la sélection des accidents sur l'écran principal s'effectue au moyen d'un crayon optique (310), lesdits moyens de visualisation auxiliaire comportant un compteur d'état (31) qui mémorise l'adresse du point de l'écran principal pointé par le crayon optique, un compteur (33) d'adressage de la mémoire auxiliaire (18), qui reçoit un signal de synchronisation provenant d'un organe de comparaison (32) entre l'adresse mémorisée dans le compteur d'état (31) et l'adresse de lecture en mémoire numérique (1 ou 2), un premier convertisseur numérique analogique auxiliaire (29) relié au compteur (33), d'adressage de la mémoire auxiliaire et apte à engendrer un signal de trame destiné au balayage vertical d'un tube cathodique de visualisation auxiliaire, un générateur de rampe, également relié au compteur (33) d'adressage de la mémoire auxiliaire et apte à engendrer un signal de balayage de ligne dudit tube cathodique de visualisation auxiliaire et un second convertisseur numérique analogique auxiliaire (19) qui reçoit les données de la mémoire auxiliaire (18).

7. Dispositif selon la revendication 6, caractérisé en ce que chacune desdites mémoires numériques (1-2) comprend une zone d'adresses réservée et que lesdits moyens de visualisation comprennent un compteur d'adresses réservées (34) servant à l'enregistrement dans ladite zone d'adresses réservée, de lignes ou portions, de lignes sélectionnées au moyen du crayon optique, des moyens (35, 24-27, 20-21), reliés audit compteur d'adresses réservées (31), de visualiser sur l'écran principal, lesdites lignes ou portions de lignes sélectionnées.

8. Dispositif selon la revendication 3, caractérisé par des moyens, déclenchés par le pointage du début d'une portion de ligne sur l'écran principal au moyen d'un crayon optique, d'inscrire dans ladite mémoire numérique (1 ou 2) un code d'effacement de ladite portion de ligne.

FIG. 1

1/2

0093032

FIG. 2

**0093032**
Numéro de la demande

Office européen
des brevets

**RAPPORT DE RECHERCHE EUROPEENNE**

EP 83 40 0719

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| Y | FR-A-2 463 953 (CHARBONNAGES DE FRANCE) * Page 5, lignes 19-26 * | 1,3 | G 06 F 15/20 |
| Y | COMPUTERS IN CARDIOLOGY, 2-4 octobre 1975, pages 15-19, Thoraxcentrum, Rotterdam, NL. M. HUBELBANK et al.: "An improved computer system for processing long-term electrocardiograms" * Page 16, colonne de gauche, lignes 40-46; page 18, colonne de gauche, lignes 1-23 * | 1,2,6 | |

DOMAINES TECHNIQUES RECHERCHES (Int. Cl. ³)

G 06 F 15/20

Le présent rapport de recherche a été établi pour toutes les revendications

| Lieu de la recherche LA HAYE | Date d'achèvement de la recherche 08-07-1983 | Examinateur BURGAUD C. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82